# EUROPÄISCHE PATENTSCHRIFT

(11) **EP 0 761 808 B1**
(45) Veröffentlichungstag und Bekanntmachung des Hinweises auf die Patenterteilung: **19.05.1999**
(21) Anmeldenummer: 96113290.9
(22) Anmeldetag: 20.08.1996
(51) Int. Cl.: C11B 9/00, A61K 7/46

(54) **Verwendung substituierter Lactone als Riechstoffe**
Use of substituted lactones as perfuming agent
Utilisation de lactones substituées comme agent parfumant

(30) Priorität: 01.09.1995 DE 19532314
(43) Veröffentlichungstag der Anmeldung: 12.03.1997
(73) Patentinhaber: HAARMANN & REIMER GMBH, D-37601 Holzminden (DE)
(72) Erfinder: Esser, Peter, Dr., 37603 Holzminden (DE); Pelzer, Ralf, Dr., 37699 Fürstenberg (DE); Völkl, Frank, New York, NY 10036 (US)
(74) Vertreter: Petrovicki, Wolfgang, Dr.

(56) Entgegenhaltungen:
- EP-A- 0 046 542
- EP-A- 0 479 222
- DE-A- 2 509 668
- FR-A- 2 211 457

## Beschreibung

Eine nicht selten unzureichende Verfügbarkeit vieler natürlicher Riechstoffkomponenten, die wechselnden Trends modischer Geschmacksrichtungen, ein steigender Bedarf an neuen Riechstoffen mit interessanten Duftnoten, aber auch eine Neubewertung vieler natürlicher und synthetischer Riechstoffe unter toxikologischen und ökologischen Aspekten erfordern neue hochqualitative Riechstoffe.

Gegenstand der Erfindung ist die Verwendung von Verbindungen der Formel 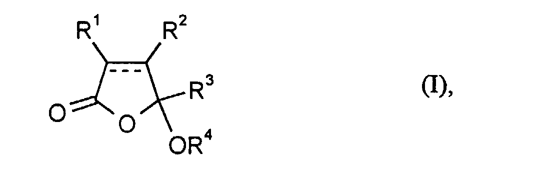 worin die gepunktete Linie eine vorhandene oder nicht vorhandene Bindung und
- R¹ bis R³: unabhängig voneinander Wasserstoff, C₁-C₁₀-Alkyl, C₅-C₁₀-Aryl oder
- R¹ und R²: zusammen oder R² und R³ zusammen C₁-C₁₀-Alkylen und
- R⁴: C₁-C₁₀-Alkyl, C₅-C₁₀-Aryl oder C₁-C₁₀-Acyl bedeuten,
als Riechstoffe.

Nach einer bevorzugten Ausführungsform werden Verbindungen I verwendet, worin
- R¹ bis R³: die oben genannte Bedeutung besitzen und
- R⁴: für C₃-C₁₀-Alkyl, insbesondere C₄-C₁₀-Alkyl, C₅-C₁₀-Aryl oder C₁-C₁₀-Acyl steht.

"Alkyl" im Sinne der Erfindung schließt Cycloalkyl ein.

Die Verbindungen I sind entweder bekannt oder können analog bekannten Verfahren hergestellt werden. So lassen sich beispielsweise 5-Alkoxy-(5H)-furan-2-one IV nach folgendem Schema herstellen:

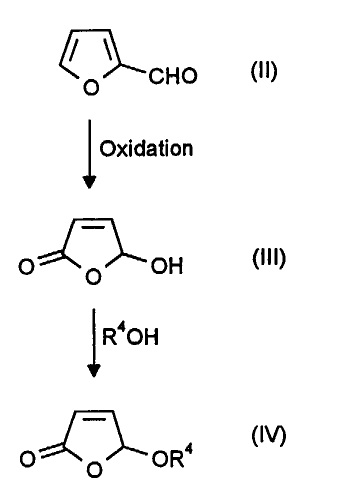

Die Umsetzung des Furfurals II zum 5-Hydroxy-(5H)-furan-2-on III kann nach verschiedenen Oxidationsverfahren erfolgen. Eine elegante Methode ist die Farbstoffsensibilisierte Photooxygenierung, wonach Furfural, gelöst in organischem Lösungsmittel, mit Licht einer Wellenlänge von 200 bis 700, vorzugsweise 400 bis 700 nm in Gegenwart eines Farbstoffs bestrahlt wird; G.O. Schenck, Liebigs Ann. Chem. 584 (1953), 156, DE-B 881 193 und 2 111 119, DE-A 43 44 163.

Geeignete Farbstoffe für die Photooxygenierung umfassen Xanthen-Farbstoffe wie Fluorescein, Eosin, Erythrosin B und Bengalrosa, Phenothiazin-Farbstoffe wie Methylenblau und Porphyrine bzw. Porphine wie Tetraphenylporphyrin, Zink-, Tetraphenylporphin und Chlorophyll. Die gewählte Konzentration an Sensibilisator hängt im wesentlichen von der Schichtdicke der bestrahlten Lösung am Einstrahlungsfenster ab; sie beträgt im allgemeinen 100 ppm bis 5 Gew.-%, bezogen auf bestrahlte Lösung.

Möchte man den Ether IV aus dem 5-Hydroxyprodukt III herstellen, kann dies durch bloßes Erhitzen in einem Überschuß des entsprechenden Alkohols oder mit etwa äquimolarer Menge Alkohol in einem als Schleppmittel für das Reaktionswasser geeigneten organischen Lösungsmittel erfolgen, gegebenenfalls in Gegenwart eines sauren Katalysators; vgl. z.B. B.L. Feringa et al., Tetrahedron 44 (1988), 7213. Ein Nachteil dieses Verfahrens ist die Notwendigkeit zweier Verfahrensschritte, bezogen auf das Ausgangsmaterial II.

Es ist allerdings auch schon darauf hingewiesen worden, daß bei Verwendung von Alkohol als Lösungsmittel bei der Photooxygenierung direkt der Ether IV erhalten werden kann. So hat offenbar die Temperatur einen wesentlichen Einfluß darauf, ob man das 5-Hydroxyprodukt III oder direkt den Ether IV erhält: Nach DE-B 2 111 119 entsteht bei einer Aufarbeitung unter 40°C das Hydroxyprodukt III, bei darüber liegenden Temperaturen direkt der dem als Lösungsmittel verwendeten Alkohol entsprechende Ether IV. Man kann also entweder in einem inerten Lösungsmittel oxidieren oder in einem Alkohol als Lösungsmittel oxidieren und bei niedriger Temperatur aufarbeiten, wenn man das Zwischenprodukt III isolieren will.

Vorzugsweise wird man also die Photooxygenierung des Furfurals II in Methanol oder Ethanol so durchführen, daß unmittelbar der Methyl- oder Ethylether IV gebildet wird. Wird der Ether eines höheren Alkohols gewünscht, so läßt sich dieser durch Umetherung erhalten. So kann man vor oder nach dem Abziehen des Methanols oder Ethanols einen höheren Alkohol zusetzen und bei erhöhter Temperatur umethern. Die Gegenwart katalytischer Mengen einer Säure, wie p-Toluolsulfonsäure, kann sowohl die Veretherung als auch die Umetherung beschleunigen.

Die 5-Acyloxyprodukte IV (R⁴ = Acyl) können ausgehend vom Hydroxyfuranon III analog zur Veresterung mit Acetylchlorid (vgl. S.H. Schroeter et al., Liebigs Ann. Chem. 697 (1966), 42, Y. Nagao et al., J. Org. Chem. 54 (1989), 5211) oder mit Acetanhydrid (V.V. Poskonin et al., J. Org. Chem. USSR 25 (1989), 1535) hergestellt werden.

Die Dihydrofuranone der Formel 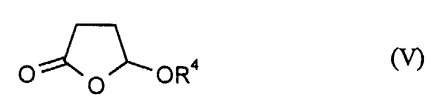 können durch Hydrierung aus den obigen Furanonen IV hergestellt werden, beispielsweise analog den von G.O. Schenck in Liebigs Ann. Chem. 584 (1953), 156 beschriebenen Verfahren.

Das Dihydrofuranon V (soweit R⁴ = Ethyl) konnte in Spuren als Weininhaltsstoff nachgewiesen werden; vgl. C.J. Muller et al., J. Agr. Food Chem. 20 (1972), 193; A.D. Webb et al., Am. J. Enol. Vitic. 27 (1976), 27; W.J. Criddle et al., Am. J. Enol. Vitic 34 (1983), 61 und J.L. Le Quéré et al., Sci. aliments 7 (1987), 93. Die Eignung dieser Verbindung wurde allerdings nicht erkannt.

In 4-Position substituierte 5-Alkoxy-(5H)-furan-2-one VIII sind sehr gut über Mannich-Reaktion aus Glyoxylsäurehydrat und den entsprechenden Aldehyden zugänglich, z.B. analog den von J.J. Bourguignon et al. in J. Org. Chem. 46, (1981), 4889 und von G. Pattenden et al. in J. Chem. Soc. (C) 1968, 1984 beschriebenen Verfahren:

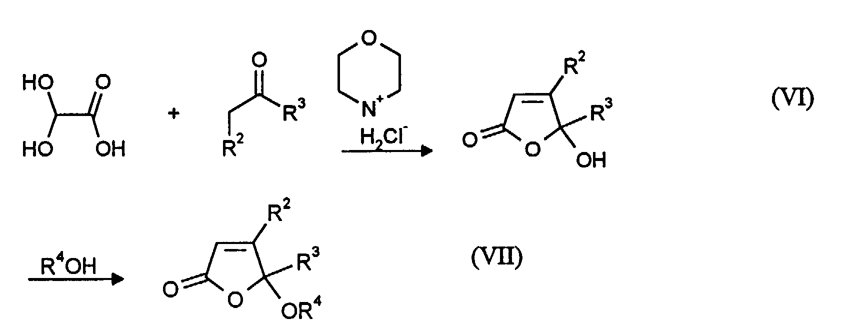

Die Veretherung von VI zu VII kann analog den weiter oben für die Veretherung von III zu IV beschriebenen Methoden erfolgen.

Auch aus cyclischen Ketonen lassen sich Furanone synthetisieren, so z.B. aus Cyclohexanon die 5,6,7,7a-Tetrahydro-7a-hydroxy-benzo-(5)-furan-2-one, vgl. J. Schreiber et al., Bull. Soc. Chim. Fr. 1973, 625, F. Bonadies et al., Gazz. Chim. Ital. 113 (1983), 421. Die entsprechenden Ether sind nach den bereits genannten Veretherungsmethoden zugänglich.

Das Hydroxyisobenzofuranon VIII kann beispielsweise nach dem Schema 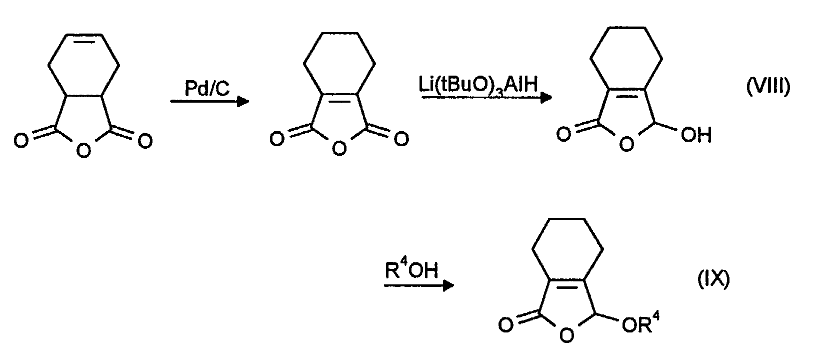 nach literaturbekannten Methoden hergestellt werden; vgl. G.E. Ficken et al., J. Chem. Soc. 1954, 3730; P. Canonne et al., Tetrahedron 44 (1988), 2903; D.W. Knight et al., J. Chem. Soc. Perkin Trans. I 1979, 62. Die Veretherung liefert dann das Produkt IX.

Die erfindungsgemäß zu verwendenden Verbindungen besitzen interessante Riechstoffeigenschaften:

Eine Tonkanote, wie sie bei Ethoxy-, Propoxy- oder Butoxy-(5H)-furan-2-on besonders ausgeprägt ist, kennzeichnet die Furanone. Je nach Strukturvariation treten weitere Aspekte hinzu oder werden dominierend. So zeigen die 4-Methyl-5-alkoxyfuranone eine starke zusätzliche Liebstöckel-Note, ein Trend, der sich allerdings feiner zu den 4-n-Propyl-5-alkoxy-furanonen fortsetzt.

Der Einfluß der 5-Alkoxy-Gruppe tritt besonders charakteristisch beim cis-Hex-3-enyl-Rest hervor, der sowohl beim Furanon als auch bei Dihydrofuranon die Tonkanote um grüne, pilzige Aspekte erweitert.

Die Fruchtigkeit ist neben den bereits benannten Tonka- und grünen Aspekten eine weitere charakteristische Note dieser Riechstoffe, die in den Acyloxyverbindungen IV und beim 5-(3-Methylbutyloxy)-(5H)-furan-2-on, dem 5-Hexyloxy-(5H)-furan-2-on und beim 5-Hexyloxy-dihydro-(3H)-furan-2-on besonders ausgeprägt ist.

Die in den Beispielen in den Tabellen 1 bis 4 dargestellten Übersichten zeigen die olfaktorischen Eigenschaften der erfindungsgemäß zu verwendenden Alkoxy- und Acyloxyfuranone.

Die erfindungsgemäß zu verwendenden Verbindungen I lassen sich gut mit anderen Riechstoffen in verschiedenen, unterschiedlichen Mengenverhältnissen zu neuartigen, interessanten Riechstoffkompositionen kombinieren, wobei die Menge 0,1 bis 50 Gew.-%, vorzugsweise 0,5 bis 10 Gew.-%, bezogen auf die ganze Komposition, beträgt.

Außer in der Feinparfümerie können derartige Kompositionen zur Parfümierung von Kosmetika wie Cremes, Lotionen, Aerosolen, Toilettenseifen, Haushaltsprodukten, wie Reinigungs- und Waschmitteln, Weichspülern, Desinfektionsmitteln und Textilbehandlungsmitteln dienen, wobei die Menge der Riechstoffkomposition 0,1 bis 40 Gew.-%, vorzugsweise 0,5 bis 20 Gew.-%, bezogen auf das ganze Produkt, beträgt.

Die Prozentangaben der nachfolgenden Beispiele beziehen sich jeweils auf das Gewicht.

### Beispiele

### Beispiel 1

Bestrahlung von Furfural mit Kunstlicht:
In einem 1 l Doppelwandgefäß mit Rührer und Gaseinleitung werden 1 mol Furfural in 1 Liter Ethanol unter Zusatz von 0,1 g Methylenblau eingefüllt. Es wird auf 20°C thermostatisiert, Sauerstoff durchgeleitet und von außen in 0,5 cm Abstand mit vier Lampen (Osram Siccatherm SL 250 W) über Nacht bis zum vollständigen Umsatz bestrahlt. Das Lösungsmittel wird unter reduziertem Druck bei einer Temperatur von nicht über 40°C abdestilliert. Das 5-Hydroxy-(5H)-furan-2-on wird im Kühlschrank auskristallisiert und mit kaltem Tetrachlorkohlenstoff ausgewaschen. Es resultieren 80 g (80 % der Theorie) an 5-Hydroxy-(5H)-furan-2-on.

Acetalisierung mit n-Propanol:
100 g (1 mol) 5-Hydroxy-(5H)-furan-2-on und 60 g (1 mol) n-Propanol werden in 500 ml Chloroform gelöst. Nach Zugabe von 0,1 g p-Toluolsulfonsäure wird so lange Wasser abgeschieden, bis die berechnete Menge von 18 ml erreicht ist. Nach Waschen mit wäßriger Natriumhydrogencarbonatlösung wird destilliert (Kp. 199°C/25 mbar). Man erhält 112 g n-Propoxy-(5H)-furan-2-on (79 % d.Th.).

### Beispiel 2

Bestrahlung von Furfural mit Sonnenlicht:
Als Reaktor diente ein Röhrenreaktor (Volumen 70 l) mit geschlossenem Kreislauf, bestehend aus einer schwenkbaren Parabolspiegelrinne (Höhe 4,5 m, Fläche 7,3 m², Aluminium-bedampftes Polytetrafluorethylen, rückseitig mit Polyesterfolie versiegelt), einem in der Brennlinie angeordneten Absorberrohr aus Glas und einem Versorgungsteil enthaltend Vorratsgefäß, Pumpe, Wärmetauscher, Gasdosierungsvorrichtung und statischen Mischer. Die Parabolspiegelrinne wurde dem Sonnenstand nachgeführt. Die Reaktionslösung wurde unter Zuführung von Sauerstoff im Kreis gepumpt und vor dem (Wieder-)eintritt in das Absorberrohr gekühlt.

Der Spiegel konzentrierte die solare Einstrahlung in das Absorberrohr auf den Faktor 20.

Der Reaktor befand sich in Spanien (37,1°, nördliche Breite).
Eine Lösung von 4,3 kg Furfural und 2,3 g Methylenblau in 30 l Ethanol wurde bei Sonnenschein unter kontinuierlicher Sauerstoffzugabe im Kreis gepumpt. Im Laufe der Reaktion wurden 2,8 g Methylenblau nachdosiert. Nach 12,6 Stunden Reaktionszeit betrug der Umsatz 99 %. Das Lösungsmittel wurde unter reduziertem Druck bei einer Wasserbadtemperatur von 70°C abdestilliert. Das so erhaltene Rohprodukt, eine Mischung aus 5-Hydroxy- und 5-Ethoxy-(5H)-furan-2-on wird ohne weitere Reinigung weiterverarbeitet.

Umetherung mit n-Hexanol:
Zu 253 g Mischung aus 5-Hydroxy- und 5-Ethoxy-(5H)-furan-2-on und 224 g n-Hexanol (äquimole Menge, bezogen auf Gehalt an Furanon), gibt man 0,1 g p-Toluolsulfonsäure. Unter reduziertem Druck wird Wasser/Ethanol abdestilliert. Nach Abdestillation der erwarteten Wasser/Ethanol-Menge von 71 g wird im Hochvakuum destilliert. Man erhält 320 g 5-Hexyloxy-(5H)-furan-2-on (Kp. 84°C/0,07 mbar) entsprechend 79 % Ausbeute.

### Beispiel 3

Bestrahlung von Furfural mit Kunstlicht:
In einem 1 Liter Doppelwandgefäß mit Rührer und Gaseinleitung werden 1 mol Furfural in 1 l Ethanol unter Zusatz von 0,1 g Methylenblau eingefüllt. Es wird auf 20°C thermostatisiert, im geschlossenen System Sauerstoff eingeleitet und von außen in 0,5 cm Abstand mit vier Lampen (Osram Siccatherm SL 250 W) über Nacht bis zum vollständigen Umsatz bestrahlt. Nachfolgend wird der Sauerstoff durch Stickstoff verdrängt und die Reaktionslösung 8 h auf Rückfluß erhitzt. Abschließend wird fraktioniert destilliert. Man erhält 286 g 5-Methoxy-(5H)-furan-2-on (Kp. 102°C/26 mbar) entsprechend 75 % Ausbeute.

**Tabelle 1**

| Verbindungen IV hergestellt analog zu den Beispielen 1 bis 3 | | | |
|---|---|---|---|
| Verbindung IV | R⁴ | Kp[°C/mbar] | Geruchsbeschreibung |
| a | Methyl | 102/26 | süß, lactonig, stechend, sauer |
| b | Ethyl | 101/13 | süß, lactonig, Cumarin |
| c | n-Propyl | 119/25 | Tonka, lactonig, fettig |
| d | iso-Propyl | 116/20 | süß, lactonig, stechend |
| e | n-Butyl | 128/19 | Tonka, lactonig, fettig |
| f | iso-Butyl | 121/20 | Tonka, fruchtig, Cocos |
| g | n-Pentyl | 65/0,05 | lactonig, fruchtig, süß, aldehydig |
| h | 1-Methylbutyl | 72/0,05 | würzig, grün, metallisch, sauer |
| i | 2-Methylbutyl | 137/25 | lactonig, fruchtig, frisch |
| j | 3-Methylbutyl | 128/20 | fruchtig, Banane, lactonig |
| k | 1,2-Dimethylpropyl | 110/10 | würzig, metallisch, frisch |
| l | cyclo-Pentyl | 76/0,34 | cumarinig, lactonig, würzig |
| m | n-Hexyl | 87/0,07 | fruchtig, lactonig, Pfirsich |
| n | cis-Hex-3-enyl | 84/0,07 | grasig, grün, Veilchen |
| o | cyclo-Hexyl | 122/5 | Tonka, süß, etherisch |
| p | n-Heptyl | 98/0,08 | fruchtig, lactonig, Pfirsich |
| q | Benzyl | 111/0,09 | schwach heliotrop |
| r | n-Octyl | 100/0,11 | fettig, frisch, metallisch |
| s | 2-Ethylhexyl | 97/0,22 | holzig, cedrig, gärig |
| t | n-Nonyl | 112/0,10 | fettig, Aldehyd, wässrig |
| u | 3,3,5-Trimethylcyclohexyl | 99/0,10 | camphrig, frisch |
| v | Phenylethyl | 123/0,06 | Rose, fruchtig, Pfirsisch |
| w | Furanmethyl | | |

### Beispiel 4

Veresterung von 5-Hydroxy-(5H)-furan-2-on:
50 g (0,5 mol) 5-Hydroxy-(5H)-furan-2-on und 65 g (0,5 mol) Propionsäureanhydrid werden mit einem Tropfen konzentrierter Schwefelsäure versetzt. Nach 2 h Erhitzen auf 100°C läßt man abkühlen und gießt auf Eiswasser. Es wird mit Essigsäureethylester extrahiert und mit 5%iger wäßriger Natriumhydrogencarbonatlösung gewaschen. Die fraktionierte Destillation liefert 67 g (43 %) 5-Propionyloxy-(5H)-furan-2-on IVc (Kp. 132°C/10 mbar).

**Tabelle 2**

| Verbindungen IV hergestellt analog zum Beispiel 4 | | | |
|---|---|---|---|
| Verbindung IV | R⁴ | Kp[°C/mbar] | Geruchsbeschreibung |
| a | Formyl | 114/12 | kulinarisch, Tonka, stechend |
| b | Acetyl | 128/14 | fruchtig, Birne, grün, sauer |
| c | Propionyl | 132/10 | fruchtig, Apfel, buttrig |

### Beispiel 5

Hydrierung von 5-Alkoxy-(5H)-furan-2-on:
276 g (1,5 mol) 5-Hexyloxy-(5H)-furan-2-on werden in 1000 ml iso-Propanol gelöst und unter Zusatz von 2 % Pd/C (5%ig) bei einem Wasserdruck von 5 bar und einer maximalen Temperatur von 40°C bis zur Beendigung der Wasserstoffaufnahme hydriert. Nach Abfiltrieren des Katalysators wird fraktioniert destilliert. Man erhält 265 g 5-Hydroxy-(3H)-dihydrofuran-2-on V g (Kp. 70°C/0,06 mbar) entsprechend 95 % Ausbeute.

**Tabelle 3**

| Verbindungen V hergestellt analog zu Beispiel 5 | | | |
|---|---|---|---|
| Verbindung V | R⁴ | Kp[°C/mbar] | Geruchsbeschreibung |
| a | Ethyl | 106/28 | käsig, gärig |
| b | n-Propyl | 96/11 | Cumarin,lactonig |
| c | n-Butyl | 126/22 | lactonig, Cumarin, süß |
| d | iso-Butyl | 106/12 | fruchtig, lactonig, Cumarin |
| e | n-Pentyl | 69/0,33 | fruchtig, lactonig, prickelnd |
| f | 1-Methylbutyl | 65/0,07 | nussig, grün, etherisch |
| g | n-Hexyl | 70/0,06 | grün, fruchtig, lactonig |
| h | cis-Hex-3-enyl | 64/0,06 | grasig, grün, metallisch |
| i | n-Octyl | 87/0,06 | fruchtig, fettig, grün |
| j | n-Nonyl | 101/0,07 | fettig, aldehydig, metallisch |
| k | 3,3,5-Trimethylcyclohexyl | 92/0,08 | Honig, süß |
| l | Cyclopentyl | 73/0,08 | Cumarin, lactonig, sahnig süß |
| m | n-Heptyl | 90/0,04 | grün, fruchtig, lactonig |
| n | Phenylethyl | | |

### Beispiel 6

Mannich-Reaktion von Glyoxylsäurehydrat und Propionaldehyd:
102 g (0,83 mol) pulverisiertes Morpholiniumchlorid und 76,4 g (0,83 mol) Glyoxylsäurehydrat werden in 400 ml Dioxan und 60 ml Wasser suspendiert. Man rührt 30 Minuten. Danach werden 53 g (0,91 mol) Propionaldehyd zudosiert, 1 h bei Raumtemperatur gerührt und 24 h unter Rückfluß erhitzt. Das Dioxan zieht man ab und versetzt noch vor der Kristallisation zügig mit Wasser. Die wässrige Phase wird über Nacht mit Ether perforiert. Die nach Trocknen und Abdestillieren des Ethers erhaltene Rohware (98,2 g) wird ohne weitere Reinigung weiterverarbeitet.

Veretherung:
Die Rohware wird mit vier Äquivalenten n-Propanol (200 g) in 500 ml Toluol unter Zusatz von 0,2 g p-Toluolsulfonsäure für 14 h unter Rückfluß erhitzt. Durch fraktionierte Destillation erhält man 104 g (80 % d.Th. über beide Stufen) 4-Methyl-5-n-propoxy-(5H)-furan-2-on VIIb (Kp. 120°C/18 mbar).

**Tabelle 4**

| Verbindungen VII hergestellt analog zu Beispiel 6 | | | | | |
|---|---|---|---|---|---|
| Verbindung VII | R² | R³ | R⁴ | Kp [°C/mbar] | Geruchsbeschreibung |
| a | Methyl | H | Methyl | 101/19 | würzig, Liebstock, Cognac |
| b | Methyl | H | n-Propyl | 120/18 | würzig, Liebstock, Cumarin |
| c | Methyl | H | n-Hexyl | 90/0,1 | fruchtig, Apfel, Birne |
| d | Methyl | H | n-Octyl | 105/0,1 | metallisch, Rose, frisch |
| e | n-Propyl | H | Methyl | 87/0,66 | grün, sauer, jasminig, Käse |
| f | n-Propyl | H | n-Propyl | 112/0,7 | brandig, Kaffee, Käse |
| g | n-Propyl | H | n-Hexyl | 100/0,09 | fruchtig, bitter |
| h | -(CH₂)₄- | | Methyl | 65/0,08 | süß, Heu, Cumarin |
| i | -(CH₂)₄- | | n-Propyl | 88/0,7 | süß, lactonig, Cumarin |

### Beispiel 7

Doppelbindungsisomerisierung:
200 g cis-1,2,3,6-Tetrahydrophthalsäureanhydrid und 5 g Pd/C (5%ig) werden 3 h unter Rühren auf 210°C erhitzt. Nach Abkühlen wird mit Wasser versetzt und aufgekocht und der Katalysator abgesaugt. Unter reduziertem Druck wird das Wasser abgezogen. Die verbleibende Kristallmasse wird wiederholt mit n-Hexan extrahiert. Man erhält 149 g rohes 3,4,5,6-Tetrahydrophthalsäureanhydrid.

Reduktion:
15,2 g (0,1 mol) rohes 3,4,5,6-Tetrahydrophthalsäureanhydrid werden unter Luftausschluß in 50 ml THF vorgelegt. Es wird auf -40°C abgekühlt und 100 ml einer 0,1 molaren Li(tBuO)₃AlH-Lösung in THF zugetropft. Über Nacht rührt man bei Raumtemperatur nach. Der Reaktionslösung werden 100 ml einer THF/10%ige Salzsäure (1/1)-Mischung zugesetzt. Nach Phasentrennung, Extraktion der wässrigen Phase und Trocknen der vereinigten organischen Phasen wird eingeengt. 4,5,6,7-Tetrahydro-3-hydroxy-1-(3H)-isobenzfuranon VIII erhält man in einer Rohausbeute von 10 g.

Veretherung:
Die 10 g VIII werden mit acht Äquivalenten n-Propanol (34 g) in 50 ml Toluol unter Zusatz von 0,1 g p-Toluolsulfonsäure für 14 h auf Rückfluß erhitzt. Nach Abkühlen wäscht man mit einer 5%igen wäßrigen Natriumhydrogencarbonatlösung. Aus Trocknung und Destillation erhält man 8,5 g (43 % d.Th. über die letzten beiden Stufen) 4,5,6,7-Tetrahydro-3-propoxy-1-(3H)-isobenzofuranon IX (Kp. 150°C/0,1 mbar). Geruchsbeschreibung: Liebstöckel, Sellerie.

### Anwendungsbeispiele

Einsatzmöglichkeiten und Wirkung der Verbindungen werden nachfolgend an einigen Beispielen demonstriert. Die nachfolgenden Kompositionen, Anwendungsbeispiele 1 bis 6, eignen sich vorzugsweise zur Feinparfümierung und zur Herstellung von Kosmetika wie Cremes, Lotionen, Aerosolen und Toilettenseifen.

### Anwendungsbeispiel 1

| **Name** | **Gew.-Teile** |
|---|---|
| Isoananat | 10 |
| Bergamottöl | 50 |
| Dihydromyrcenol | 300 |
| Lilial | 50 |
| Linalool | 20 |
| Geraniumöl Bourbon | 15 |
| Methyldihydrojasmonat | 100 |
| Eugenol | 50 |
| Zimtrindenöl Ceylon | 2 |
| Cedernholzöl Chin. | 20 |
| Vertofix coeur | 100 |
| Hexahydroiraldein | 50 |
| 1,2,3,4,5,6,7,8-Octahydro-2,3,8,8-tetramethyl-2-acetonaphton | 150 |
| Evernyl | 10 |
| Ambroxid | 5 |
| Diethylphthalat | 58 |
| 5-Propoxy-(5H)-furan-2-on | 10 |
| **Summe** | **1000** |

Das-5-Propoxy-(5H)-furan-2-on unterstreicht in dieser Komposition die Tonkaaspekte. Es hat einen angenehm harmonisierenden Einfluß und trägt zu einer guten Fixierung bei.

### Anwendungsbeispiel 2

| **Name** | **Gew.-Teile** |
|---|---|
| Bergamottöl | 200 |
| Dihydromyrcenol | 20 |
| Linalylacetat | 40 |
| Citronenöl | 50 |
| Lyral | 50 |
| Methyldihydrojasmonat | 15 |
| Isoamylsalicylat | 15 |
| Bayöl | 5 |
| Cumarin | 10 |
| Vertofix coeur | 120 |
| 1,2,3,4,5,6,7,8-Octahydro-2,3,8,8-tetramethyl-2-acetonaphton | 220 |
| Timberol | 20 |
| Patchouliöl | 15 |
| Sandel 80 | 10 |
| Tonalid | 100 |
| Chromanolid 50 % (in Ethanol) | 100 |
| 5-cis-Hex-3-enyloxy-(5H)-furan-2-on | 10 |
| **Summe** | **1000** |

5-cis-Hex-3-enyloxy-(5H)-furan-2-on gibt diesem Akkord eine besonders ausdrucksvolle grüne Veilchennote, die zur Strahlung des Duftes im Raum beiträgt.

### Anwendungsbeispiel 3

| **Name** | **Gew.-Teile** |
|---|---|
| Liffarome | 5 |
| Allylamylglycolat | 5 |
| Bergamottöl | 90 |
| γ-Nonalacton | 5 |
| Lilial | 80 |
| Lyral | 80 |
| Linalool | 150 |
| Phenylethylalkohol | 50 |
| Citronellol | 20 |
| Benzylacetat | 25 |
| α-Hexylzimtaldehyd | 100 |
| cis-Hex-3-enylsalicylat | 30 |
| γ-Iraldein | 50 |
| Cedramber | 20 |
| 1,2,3,4,5,6,7,8-Octahydro-2,3,8,8-tetramethyl-2-acetonaphton | 30 |
| Sandalore | 40 |
| Mousse C abs., 50 % in Ethanol | 20 |
| Ethylenbrassylat | 50 |
| Cyclopentadecanolid supra | 30 |
| Chromanolid, 50 % in Ethanol | 100 |
| 5-Hexyloxy-(5H)-furan-2-on | 20 |
| **Summe** | **1000** |

In diesem blumigen Akkord unterstützt 5-Hexyloxy-furan-2-(5H)-on das fruchtige Bouquet und verleiht ihm eine besondere Fülle.

### Anwendungsbeispiel 4

| **Name** | **Gew.-Teile** |
|---|---|
| Citrophoral supra | 60 |
| Styrolylacetat | 50 |
| Tamarine Base | 80 |
| 1-Methylacetat | 20 |
| γ-Decalacton | 60 |
| γ-Undecalacton | 20 |
| Diethylmalonat | 40 |
| Pfirsich Base | 60 |
| Buccoblätteröl coeur | 5 |
| Linalooloxid | 20 |
| Linalool | 80 |
| Dimethylbenzylcarbinylbutyrat | 40 |
| Benzylacetat | 50 |
| α-Hexylzimtaldehyd | 100 |
| α-Amylzimtaldehyd | 80 |
| Agrumex | 20 |
| Cyclopentadecanolid supra | 15 |
| Tonalid | 60 |
| Chromanolid, 50 % in Ethanol | 20 |
| 5-Hexyloxy-(3H)-dihydrofuran-2-on | 40 |
| **Summe** | **1000** |

Die Pfirsich/Apfel-Gesamtnote dieser Komposition erhält durch das 5-Hexyloxydihydro-(3H)-furan-2-on wesentlich mehr Fülle und wird im fruchtigen Gesamteindruck deutlich gestärkt.

### Anwendungsbeispiel 5

| **Name** | **Gew.-Teile** |
|---|---|
| Lavandinöl | 50 |
| γ-Nonalacton | 300 |
| γ-Decalacton | 10 |
| Diacetyl | 50 |
| Phenylethylalkohol | 90 |
| Heliotropin | 80 |
| Anisaldehyd | 5 |
| Zimtalkohol | 50 |
| Cumarin | 200 |
| Dipropylenglykol | 5 |
| Vanille-Base | 150 |
| 5-Cyclopentyloxy-(5H)-furan-2-on | 10 |
| **Summe** | **1000** |

Das 5-Cyclopentyloxy-(5H)-furan-2-on mit seiner Gesamtbeschreibung Tonka, Kokos und Süße ergänzt diese Kokos/Karamel-Geruchsnote. Es resultiert ein insgesamt natürlicher Eindruck.

### Anwendungsbeispiel 6

| **Name** | **Gew.-Teile** |
|---|---|
| Liffarome | 2 |
| cis, trans-Hex-3-enylacetat | 2 |
| Phenylacetaldehydglycerinacetal | 30 |
| Styrolylacetat | 2 |
| Cyclamenaldehyd | 5 |
| Florol | 40 |
| Lyral | 300 |
| Hydroxycitronellal | 150 |
| Farnesol | 10 |
| Linalool | 20 |
| Phenylethylalkohol | 150 |
| Citrolellol | 20 |
| Geranylacetat | 5 |
| Methyldihydrojasmonat | 230 |
| Hexylbenzoat | 10 |
| Indol | 2 |
| Diethylphthalat | 12 |
| 5-cis-Hex-3-enyloxy-dihydro-(5H)-furan-2-on | 10 |
| **Summe** | **1000** |

Der Einsatz von 5-cis-Hex-3-enyloxy-dihydrofuranon in diesem Maiglöckchen-Akkord unterstützt insbesondere die natürlich wirkende Grünnote.

## Patentansprüche

1. Verwendung von Verbindungen der Formel 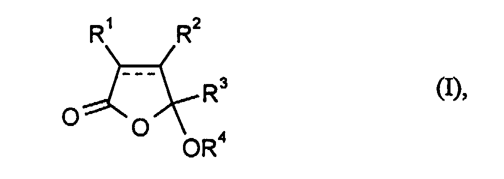 worin die gepunktete Linie eine vorhandene oder nicht vorhandene Bindung und
R¹ bis R³ unabhängig voneinander Wasserstoff, C₁-C₁₀-Alkyl, C₅-C₁₀-Aryl oder
R¹ und R² zusammen oder R² und R³ zusammen C₁-C₁₀-Alkylen und
R⁴ C₁-C₁₀-Alkyl, C₅-C₁₀-Aryl oder C₁-C₁₀-Acyl bedeuten,
als Riechstoffe.

2. Verwendung nach Anspruch 1, wobei die gepunktete Linie eine Bindung bedeutet und R¹ bis R³ für Wasserstoff und R⁴ für C₁-C₁₀-Alkyl stehen.

3. Verwendung nach Anspruch 1, wobei die gepunktete Linie eine Bindung bedeutet und R¹ bis R³ für Wasserstoff und R⁴ für C₁-C₁₀-Acyl stehen.

4. Verwendung nach Anspruch 1, wobei die gepunktete Linie keine Bindung bedeutet und R¹ bis R³ für Wasserstoff und R⁴ für C₁-C₁₀-Alkyl stehen.

5. Verwendung nach Anspruch 1, wobei die gepunktete Linie eine Bindung bedeutet und R¹ bis R³ für Wasserstoff, R² für C₁-C₁₀-Alkyl (oder R² und R³ zusammen für C₁-C₁₀-Alkylen) und R⁴ für C₁-C₁₀-Alkyl stehen.

## Claims

1. Use of compounds corresponding to the formula 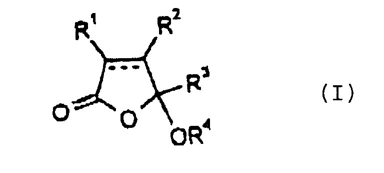 wherein the dotted line denotes a bond which may or may not be present and
R¹ to R³ independently of one another denote hydrogen, C₁-C₁₀-alkyl, C₅-C₁₀-aryl or
R¹ and R² together or R² and R³ together denote C₁-C₁₀-alkylene and
R⁴ denotes C₁-C₁₀-alkyl, C₅-C₁₀-aryl or C₁-C₁₀-acyl,
as perfumes.

2. Use according to claim 1, wherein the dotted line denotes a bond and R¹ to R³ represent hydrogen and R⁴ represents C₁-C₁₀-alkyl.

3. Use according to claim 1, wherein the dotted line denotes a bond and R¹ to R³ represent hydrogen and R⁴ represents C₁-C₁₀-acyl.

4. Use according to claim 1, wherein the dotted line indicates no bond and R¹ to R³ represent hydrogen and R⁴ represents C₁-C₁₀-alkyl.

5. Use according to claim 1, wherein the dotted line denotes a bond and R¹ to R³ represent hydrogen, R² represents C₁-C₁₀-alkyl (or R² and R³ together represent C₁-C₁₀-alkylene) and R⁴ represents C₁-C₁₀-alkyl.

## Revendications

1. Utilisation de composés de la formule 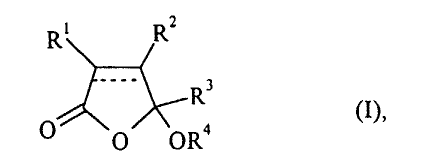 dans laquelle la ligne en pointillés présente une liaison existante ou inexistante et
R¹ à R³ représentent indépendamment un atome d'hydrogène, un groupe alkyle en C₁-C₁₀, un groupe aryle en C₅-C₁₀ ou
R¹ et R² ensemble ou R² et R³ ensemble représentent un groupe alkylène en C₁-C₁₀ et
R⁴ représente un groupe alkyle en C₁-C₁₀, un groupe aryle en C₅-C₁₀ ou un groupe acyle en C₁-C₁₀,
comme agents parfumants.

2. Utilisation selon la revendication 1, la ligne en pointillés représentant une liaison et R¹ à R³ représentant un atome d'hydrogène et R⁴ représentant un groupe alkyle en C₁-C₁₀.

3. Utilisation selon la revendication 1, la ligne en pointillés représentant une liaison et R¹ à R³ représentant un atome d'hydrogène et R⁴ représentant un groupe acyle en C₁-C₁₀.

4. Utilisation selon la revendication 1, la ligne en pointillés ne représentant pas de liaison et R¹ à R³ représentant un atome d'hydrogène et R⁴ représentant un groupe alkyle en C₁-C₁₀.

5. Utilisation selon la revendication 1, la ligne en pointillés représentant une liaison et R¹ à R³ représentant un atome d'hydrogène, R² représentant un groupe alkyle en C₁-C₁₀ (ou R² et R³ ensemble un groupe alkylène en C₁-C₁₀) et R⁴ représentant un groupe alkyle en C₁-C₁₀.
